Europäisches Patentamt

European Patent Office

Office européen des brevets

(19)

(11) Veröffentlichungsnummer: **0 104 452**

**B1**

(12) # EUROPÄISCHE PATENTSCHRIFT

(45) Veröffentlichungstag der Patentschrift:
26.11.86

(21) Anmeldenummer: 83108402.5

(22) Anmeldetag: 26.08.83

(51) Int. Cl.⁴: **A 61 N 1/362,** A 61 N 1/08,
A 61 B 5/04

(54) Synchronisierbarer Herzschrittmacher mit Störerkennungsschaltung.

(30) Priorität: 01.09.82 DE 3232478

(43) Veröffentlichungstag der Anmeldung:
04.04.84 Patentblatt 84/14

(45) Bekanntmachung des Hinweises auf die Patenterteilung:
26.11.86 Patentblatt 86/48

(84) Benannte Vertragsstaaten:
DE FR GB IT NL SE

(56) Entgegenhaltungen:
AT-B-322 095
AT-B-364 072
US-A-3 833 005
US-A-3 985 142

(73) Patentinhaber: Irnich, Werner, Prof. Dr.- Ing.,
Birkenweg 60, D-6301 Wettenberg 3 (DE)

(72) Erfinder: Irnich, Werner, Prof. Dr.- Ing.,
Birkenweg 60, D-6301 Wettenberg 3 (DE)

(74) Vertreter: Biermann, Wilhelm, Dr.- Ing.,
Morillenhang 39, D-5100 Aachen (DE)

**Beschreibung**

Die Erfindung betrifft einen synchronisierbaren Herzschrittmacher mit einem Zeitgeberkreis und einer Störerkennungsschaltung, die eine Untersuchungsstufe und eine nachfolgende Diskriminatorstufe umfaßt, in der das Ausgangssignal der Untersuchungsstufe nach einem vorgegebenen, etwa der Zeitdauer des QRS-Komplexes des Herzaktionssignals entsprechenden Zeitintervall daraufhin geprüft wird, ob es noch vorhanden ist, wobei als Ergebnis dieser Prüfung entweder das Signal als Herzsignal erkannt und an den Zeitgeberkreis weitergeleitet oder als Störsignal erkannt und unwirksam gemacht wird.

Bei einem bekannten Herzschrittmacher dieser Art (US-PS 3 985 142) umfaßt die Untersuchungsstufe in der Störerkennungsschaltung eine Komparatorschaltung, in der das Eingangssignal mit einem Referenzsignal verglichen wird, wobei das Ausgangssignal der Komparatorschaltung vom Ergebnis dieses Vergleichs abhängt. Diese bekannte Störerkennungsschaltung ist jedoch nicht in der Lage, symmetrische Störsignale als solche zu erkennen, wenn diese in ihrer Zeitdauer den Herzaktionssignalen entsprechen bzw. kürzer sind als die Refraktärzeit.

Der Erfindung liegt die Aufgabe zugrunde, die Störerkennungsschaltung in einem Herzschrittmacher der genannten Art dahingehend zu verbessern, daß zusätzlich symmetrische Störsignale, die ansonsten in ihrer Zeitdauer den Herzaktionssignalen entsprechen bzw. kürzer sind als die Zeitdauer des QRS-Komplexes des Herzaktionssignals (Refraktärzeit), als solche erkannt und unschädlich gemacht werden.

Erfindungsgemäße wird diese Aufgabe dadurch gelöst, daß die am Eingang der Störerkennungsschaltung anliegende Spannung in einem Differenzierer differenziert, und die differenzierte Spannung der Untersuchungsstufe zugeführt wird, in der der positive und der negative Spitzenwert jedes differenzierten Signals gespeichert und aus diesen Spitzenwerten die Summe gebildet wird, wobei eine negative Summenspannung bestimmter Größe ein Herzaktionssignal repräsentiert.

Die erfindungsgemäße Störerkennungsschaltung ist deswegen besonders wirkungsvoll, weil sie im Gegensatz zu den bekannten Störerkennungsschaltungen die typische Struktur eines intrakardialen Signals, gleichgültig ob es atrial oder ventrikulär abgeleitet wird, berücksichtigt. Die typische Struktur eines unipolar abgeleiteten Herzsignals, bei dem sich eine Elektrode unmittelbar an der Herzwand befindet, während die andere demgegenüber weit entfernt liegt, zeichnet sich dadurch aus, daß es mit einer mehr oder weniger großen positiven Anfangszacke beginnt, dann in sehr kurzer Zeit zu einer negativen Zacke abfällt, der wiederum ein positiver Anstieg mit verminderter Steilheit folgt. Die sich daran anschließende plumpere Welle kann unterschiedliche Formen annehmen; sie kann relativ groß in ihrem positiven Anteil sein, vor allem bei Erstimplantation, jedoch kann dieser Teil auch ganz fehlen, vor allem bei lange liegenden Elektroden. In jedem Fall ergibt sich zum Ende der Herzaktion wieder eine plumpe Welle, die als T-Welle bezeichnet wird.

Die Abfallzeit von dem Spitzenwert der positiven Anfangszacke zum Spitzenwert der negativen Zacke beträgt nach eigenen Messungen zwischen 0,5 und 10,9 ms, d.h. im Mittel 4,6 ms, während die sich daran anschließende Anstiegszeit bis zum Spitzenwert der folgenden Welle zwischen 3,8 und 35 ms beträgt. Das bedeutet, daß zwischen der Abfallzeit und der Anstiegszeit ein Verhältnis von etwa 1:4 besteht. Dem Unterschied zwischen Abfalls- und Anstiegszeit entspricht der Unterschied in der negativen und der positiven Flankensteilheit des Signals, die sich umgekehrt wie etwa 4:1 verhalten.

Die erfindungsgemäße Störerkennungsschaltung baut auf der Erkenntnis auf, daß weder exogene noch endogene Störsignale, d.h. körpereigene Signale z.B. aus der jeweils anderen Herzkammer oder Muskelsignale, die genannte charakterische Eigenschaft der in dem genannten Verhältnis zueinander liegenden Flankensteilheiten der Herzsignale aufweisen. Sie benutzt dieses Kriterium für die Erkennung von Störsignalen, indem sie die Signale daraufhin untersucht, ob sie dem genannten Verhältnis der Flankensteilheiten entsprechen. Damit ist die Störerkennungsschaltung in der Lage, auch Störsignale als solche zu erkennen, deren Zeitdauer derjenigen der Herzaktionssignale entspricht bzw. kürzer ist als die Zeitdauer des QRS-Komplexes (Refraktärzeit) des Herzaktionssignals.

Weitere Vorteile und zweckmäßige Ausgestaltungen und Weiterbildungen der Erfindung ergeben sich aus den Unteransprüchen und aus der nachfolgenden Beschreibung verschiedener Ausführungsbeispiele der Erfindung anhand der Zeichnungen.

Von den Zeichnungen zeigt

Fig. 1 das Blockschaltbild der Störerkennungsschaltung des erfindungsgemäßen Herzschrittmachers;

Fig. 2 das Schaltbild einer ersten Ausführungsform der Störerkennungsschaltung gemäß Fig. 1;

Fig. 3 bis 9 eine andere Ausführungsform der Störerkennungsschaltung gemäß Fig 1, wobei die einzelnen Figuren die einzelnen Blöcke des Blockschaltbildes nach Fig. 1 darstellen.

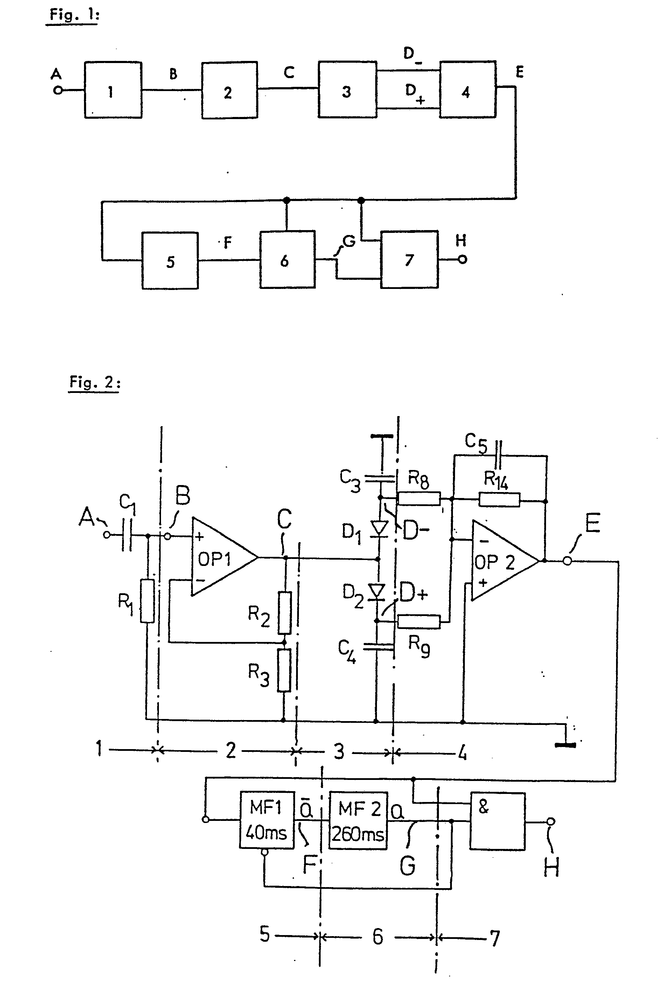

Die elektronische Störerkennungsschaltung und ihre charakteristische Wirkungsweise werden zunächst anhand des in Fig. 1 dargestellten Blockschaltbildes erläutert.

Am Eingang A der Störerkennungsschaltung befindet sich ein Differenzierer 1, der die positive

und die negative Flankensteilheit der Signale elektronisch erfaßt.

Dieser Differenzierer 1 wandelt die Steilheit der ansteigenden Flanke des Signals in eine positive Spannung und die Steilheit der abfallenden Flanke des Signals in eine negative Spannung um.

Dem Differenzierer 1 schließt sich die sogenannte Untersuchungsstufe an, die den Vorverstärker 2, den Spitzenwertspeicher 3 und die Signalformierstufe 4 umfaßt. Die am Ausgang des Differenzierers 1 im Punkt B anliegende Spannung wird in dem Vorverstärker 2 soweit verstärkt, daß an dessen Ausgang C eine Spannungsamplitude zur Verfügung steht, die sich für die Weiterverarbeitung eignet. In dem Spitzenwertspeicher 3 wird sowohl das der negativen Steilheit als auch das der positiven Steilheit entsprechende Spannungssignal gespeichert, wobei die entsprechenden Ausgangssignale D- und D+ für eine ausreichend lange Zeit zur Verfügung stehen. Die Signalformierschaltung 4 schließlich hat die Aufgabe, die der negativen und der positiven Steilheit entsprechenden Signale D- und D+ zu addieren, die so gebildete Summenspannung zu verstärken, und die verstärkte Summenspannung in einer Komparatorschaltung auf ihre Größe zu untersuchen. Ist das Summensignal gleich oder größer als ein Vergleichssignal, dann schaltet die Komparatorschaltung von einem niedrigen Wert auf einen hohen Wert um, so daß am Ausgang E der Signalformierstufe 4 entweder ein niedriger oder ein hoher Spannungswert anliegt. An dieser Stelle erfolgt der Übergang von der analogen Signalverarbeitung zur digitalen Technik.

Das am Ausgang E der Signalformierschaltung anliegende Signal wird an die Diskriminatorstufe weitergeleitet, die aus dem Verzögerungsglied 5, der Refraktärschaltung 6 und der Koinzidenzschaltung 7 besteht. Das Verzögerungsglied 5 schaltet nach Ablauf einer Verzögerungszeit, die etwa der Dauer des QRS-Komplexes entspricht, am Ausgang F von niedrigem Spannungspegel auf einen hohen Spannungspegel um. Dadurch spricht in der Refraktärschaltung 6 für eine bestimmte Zeitdauer zwischen 200 und 300 ms ein Zeitglied an. Der Ausgang G der Refraktärschaltung 6 wird auf einen der Eingänge der Koinzidenzschaltung 7 gegeben. Wenn gleichzeitig an den beiden mit den Ausgängen E und G verbundenen Eingängen der Koinzidenzschaltung 7 Signale mit hohem Pegel anliegen, führt das am Ausgang H der Koinzidenzschaltung 7 zu einem Signal mit hohem Pegel, das eine Herzaktion charakterisiert.

Falls das am Eingang A anliegende Signal kein Herzsignal ist, sondern ein Störsignal, beispielsweise ein symmetrisches Störsignal oder ein Signal mit symmetrischer Flankensteilheit ist, wie es etwa durch Doppelweg-Gleichrichter hervorgerufen wird, dann ist die Summenspannung in der Signalformierschaltung 4 gleich Null oder nahezu gleich Null. Infolgedessen hat bei solchen Signalen die Spannung am Ausgang E der Signalformiererschaltung 4 einen niedrigen Pegel. In diesem Fall liegt an den beiden Eingängen der Koinzidenzschaltung 7 nicht gleichzeitig ein hohes Signal, eine "Eins" an, so daß solche Signale als Störsignale erkannt und verworfen werden. Da auch Herzsignale der anderen Herzkammer und Muskelsignale sehr viel symmetrischer in ihrer Flankensteilheit sind, werden bei geeigneter Verstärkungsschaltung auch solche Signale wie Störsignale unterdrückt.

Das Blockdiagramm nach Fig. 1 kann beispielsweise durch die Schaltung nach Fig. 2 realisiert werden. Das am Eingang A anliegende Signal wird in dem aus dem Kondensator $C_1$ und dem Widerstand $R_1$ bestehenden Differenzierer differenziert, und der Ausgang B des Differenzierers auf den positiven Eingang des Operationsverstärkers $OP_1$ gegeben. Das Verhältnis der Rückkoppel-Widerstände $R_2$ zu $R_3$, rückgekoppelt auf den negativen Eingang von $OP_1$, bewirkt die Verstärkung des differenzierten Eingangssignals derart, daß das Steilheitssignal auf einen Pegel von etwa 0,5 bis 1 V gebracht wird. Über die beiden Dioden $D_1$ und $D_2$ werden die Kondensatoren $C_3$ und $C_4$ aufgeladen, wobei das positive Steilheits-Maximum D+ an $C_4$, und das negative Steilheitsmaximum D- an $C_3$ anstehen. Der Operationsverstärker $OP_2$ stellt zusammen mit den Widerständen $R_8$ und $R_9$ sowie $R_{14}$ im Rückkoppelzweig einen verstärkenden Addierer dar, der am Ausgang E die Summe aus dem positiven und dem negativen Steilheitsmaximum wiedergibt. Auf diese Weise wird, abgesehen von einem kurzzeitigen Anfangssignal, am Verstärkerausgang E bei jeder symmetrischen Störung oder Störung mit symmetrischer Steilheit das Summen-Signal zu Null werden. Die Kondensatoren $C_3$ und $C_4$ werden gleichzeitig über die Widerstände $R_8$ und $R_9$ langsam entsprechend ihrer Zeitkonstante entladen, so daß bei im Herzrhythmus auftretenden Signalen die als Speicher wirkenden Kondensatoren $C_3$ und $C_4$ praktisch immer wieder entladen werden. Auf diese Weise werden die Steilheiten eines jeden Herzsignals gespeichert und der Steilheitsvergleich durchgeführt. Bei schnell aufeinanderfolgenden Signalen, wie sie typisch für 50 oder 60 Hz-Störungen sind, haben die beiden Kondensatoren nicht die Zeit, sich nennenswert zu entladen. In dieser Situation wird also jeweils der Maximalwert der Steilheit gespeichert.

Das Signal am Ausgang E wird in der nachfolgenden Diskriminator-Schaltung daraufhin untersucht, ob es sich um ein Signal mit unsymmetrischen Steilheiten handelt oder nicht. Dazu wird durch jedes Signal am Ausgang E ein monostabiler Multivibrator MF1 im Sinne eines Verzögerungsgliedes mit etwa 40 ms Verzögerungszeit angesteuert. Der invertierte Ausgang $\bar{Q}$ dieses Multivibrators MF1 steuert einen zweiten monostabilen Multivibrator MF2 mit einer Zeit von etwa 260 ms über den dynamischen Eingang an. Erhöht sich dessen Ausgang Q vom logischen "Null" auf "Eins", so wird der Ausgang des folgenden AND-Gatters 7 ein "Eins"-Signal dann annehmen, wenn bis zum Ende der Verzögerungszeit von MF1 das Ausgangssignal E von OP2 positiv geblieben ist. Ein solcher Zustand ist nur möglich, wenn am Eingang A der gesamten Schaltung ein Signal angelegen hat, dessen Betrag der negativen Steilheit deutlich größer ist als der der positiven Steilheit, was eben typisch für intrakardiale Herzsignale ist. Demgegenüber wird jedes einmalige Signal mit überwiegend positiver Steilheit, darüberhinaus jede gepulste oder amplitudenmodulierte Störung durch die Addition vom Maximum und Minimum der Steilheit am Ausgang E des Verstärkers OP2 immer zu einem Signal führen, das kürzer als die Zeit des monostabilen Multivibrators MF1 ist.

Das am Ausgang H anstehende Signal, das immer nur dann erzeugt wird, wenn ein Signal wie ein intrakardiales Herzsignal die entsprechenden Steilheits-Kriterien aufweist, steuert den üblichen Zeitgeberkreis an, in dem aufgrund dieses Signals entweder das Erwartungs-Interval eines Bedarfs-Schrittmachers neu gestartet oder aber, wie im Falle der vorhofgesteuerten Schrittmacher, ein Verzögerungsglied entsprechend der AV-Überleitungszeit aktiviert wird.

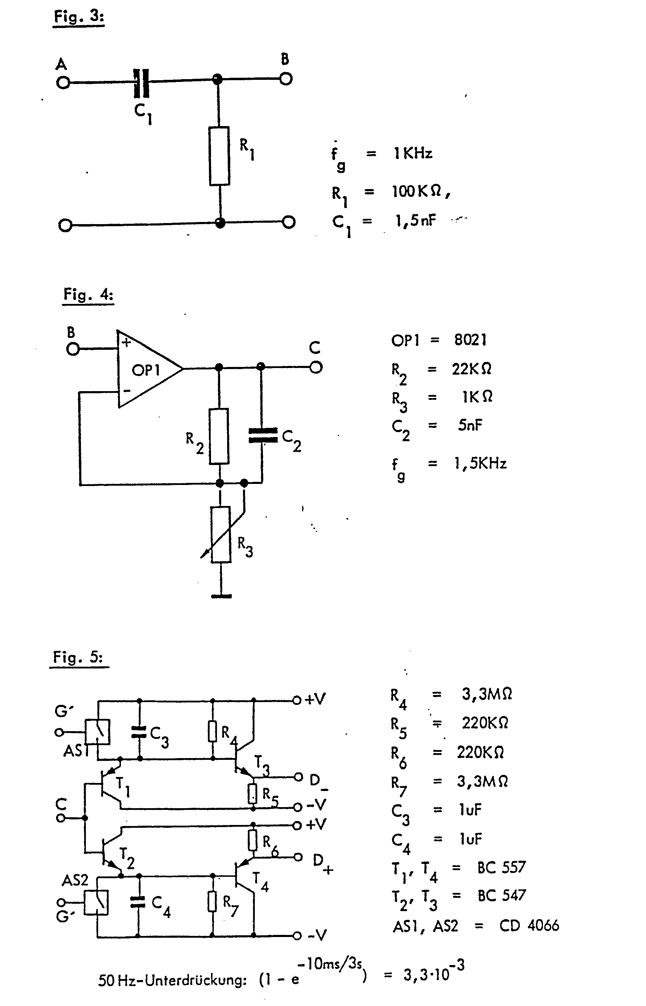

In den Figuren 3 bis 9 wird eine erweiterte Ausführungsform der erfindungsgemäßen Schaltung anhand der einzelnen Blöcke des Block-Diagramms nach Fig. 1 im einzelnen dargestellt, wobei zusätzliche oder erklärende Merkmale erläutert werden. Der Differenzierer 1 bestehend aus dem RC-Glied muß so bemessen werden, daß er eine Abfallzeit von 0,5 ms noch einigermaßen ungedämpft differenzierend wiedergibt. Einer Anstiegszeit von 0,5 ms entspricht eine Grenzfrequenz $f_g$ des Differenzierers von 1 KHz.

Beim Vorverstärker 2 entsprechend Fig. 4 wird einmal der Widerstand $R_3$ als Potentiometer ausgeführt, um die Verstärkung variabel zu gestalten, zum anderen wird über den Rückkoppelkondensator $C_2$ eine frequenzabhängige Verstärkung eingeführt. Wählt man als Grenzfrequenz $f_g$ dieses Tiefpasses 1,5 KHz, ergibt sich als Zeitkonstante des aus $R_2$ und $C_2$ bestehenden Kreises der Wert von 0,1 ms.

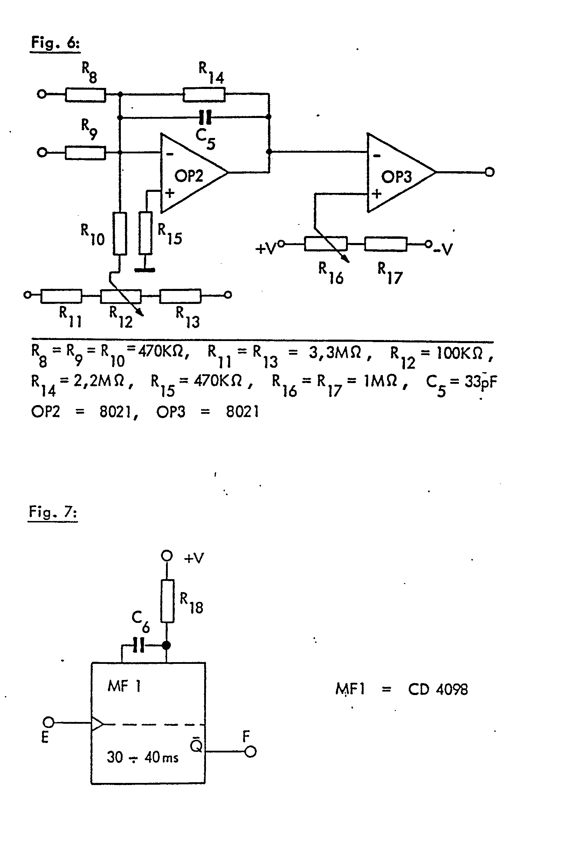

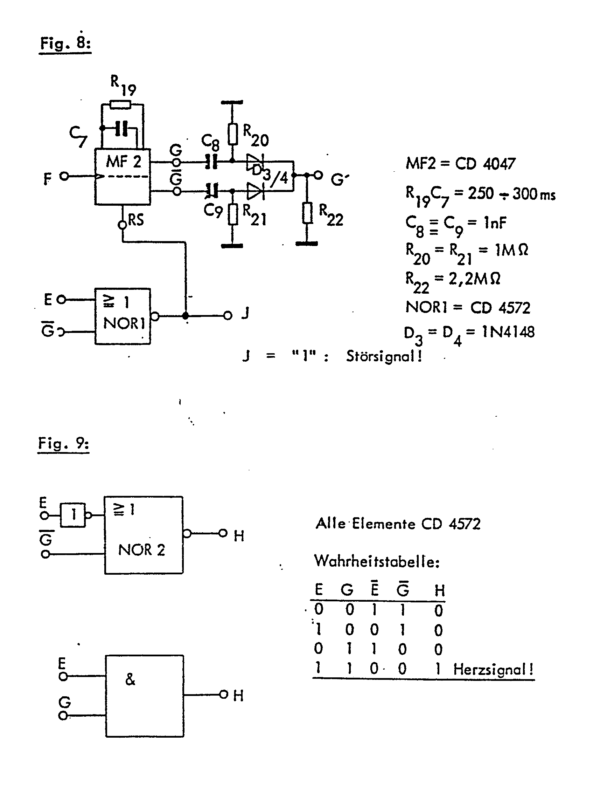

In Fig. 5 wird der Spitzenwert-Speicher 3 dargestellt, wie er sich zur Erfüllung der von ihm geforderten Aufgaben als optimal erwiesen hat. Statt der Dioden $D_1$ und $D_2$ in Fig. 2 werden die Basis-Emitter-Dioden von Transistoren $T_1$ und $T_2$ in npn- und pnp-Technik an den Ausgang C vom Vorverstärker 2 angeschlossen. Hierdurch wird der Ausgang des Vorverstärkers strommäßig entlastet, die Transistoren wirken wie Emitter-Folger. Um das Potential an den beiden Kondensatoren $C_3$ und $C_4$ belastungsunabhängig von der darauffolgenden Stufe (Signalformiererstufe) 4 zu machen, werden

wiederum zwei Transistoren $T_3$ und $T_4$ im Sinne eines Emitter-Folgers angeschlossen, wobei jedoch deren Polarität umgekehrt zu der vorangehenden Transistor-Stufe ist. Dadurch wird nicht nur eine Entkoppelung erreicht, sondern auch ein Potential an D+ oder D-geschaffen, das identisch mit dem jeweiligen Maximum oder Minimum am Ausgang des Vorverstärkers 2 in C ist (gegenseitige Kompensation der Schleusenspannungen). Die Kondensatoren $C_3$ und $C_4$ werden langsam mit einer Zeitkonstante von 3 s über die Widerstände $R_4$ bzw. $R_7$ entladen. Sie können aber auch über die analogen Schalter AS1 bzw. AS2 schnell entsprechend dem Steuersignal G', das aus der Refraktärschaltung nach Fig. 8 abgeleitet wird, entladen werden.

In Fig. 6 ist die Signalformierschaltung 4 dargestellt. Sie besteht wiederum aus einem Addierer, allerdings jetzt mit Offset-Abgleich, und einem nachgeschalteten Komparator, bestehend aus dem Operationsverstärker OP3, dessen negativer Eingang mit einem Spannungsteiler gebildet aus den Widerständen $R_{16}$ und $R_{17}$ verbunden ist. Immer dann, wenn die Ausgangsspannung von OP2 den Spannungspegel am Potentiometer $R_{17}$ übersteigt, schaltet der Ausgang E von OP3 von niedrigem auf hohes Potential um. Zur Bedämpfung hochfrequenter Spannungen großer Amplitude wird dem Rückkoppel-Widerstand $R_{14}$ von OP2 ein Kondensator $C_5$ parallelgeschaltet, der die verstärkenden Eigenschaften der Addierstufe auf einen Frequenzbereich bis 1,5 KHz begrenzt.

Das Verzögerungsglied 5 in Fig. 7 bedarf keiner besonderen Erläuterung. Auf ein Eingangssignal E hin wird über den dynamischen Eingang von MF1 der Multivibrator aktiviert und verbleibt für eine Zeit, die zweckmäßigerweise zwischen 30 und 40 ms gewählt wird, im angeregten Zustand. Das Ausgangssignal F wird vom invertierenden Ausgang Q̄ gewonnen.

Die Refraktärschaltung 6 nach Fig. 8 weist zusätzliche Elemente auf, wie sie bisher bei Störerkennungs-Schaltungen nicht verwendet wurden. Wird auf ein Eingangssignal F hin der monostabile Multivibrator MF2 aktiviert, so wird gleichzeitig über ein NOR-Gatter NOR1 die Koinzidenz überprüft, ob während der Refraktärzeit von MF2 am Ausgang E des Signal-Formierers 4 ein Signal mit hohem oder mit niedrigem Potential ansteht. Man sieht, daß immer dann der Reset-Eingang RS mit einer "Eins" aktiviert wird, wenn eine Störung den Anlaß für eine Aktivierung des Verzögerungsgliedes 5 und folglich auch der Refraktärschaltung 6 gewesen ist (E und Ḡ ist dann "Null"). Die Ausgänge G und Ḡ werden über die RC-Glieder $C_8$, $R_{20}$ und $C_9$, $R_{21}$ auf die Dioden D3 und D4 am Widerstand $R_{22}$ addiert. An G' liegt also immer ein positiver Impuls zu Beginn und am Ende der Refraktärzeit an, der benutzt wird, um in dem Spitzenwert-Speicher die Kondensatoren $C_3$ und $C_4$ über die Analog-Schalter AS1 und AS2 schnell zu entladen. Die Refraktärschaltung 6 nach Fig. 8 hat also die Eigenschaft, nur nach einem als Herzsignal identifizierten Signal eine Refraktärzeit voller Länge Wahrnehmungspause) in den Signalablauf einzubringen. Im Falle einer Störung wird die Refraktärschaltung unmittelbar nach Aktivierung wieder zurückgesetzt, so daß ein tatsächliches Herzsignal nicht Gefahr läuft, übersehen zu werden.

Die Koinzidenzschaltung 7 nach Fig. 9 kann entweder im Sinne einer AND-Schaltung realisiert werden, die immer dann an ihrem Ausgang H ein Signal hohen Potentials abgibt, wenn sowohl das Signal E am Ausgang des Signal-Konditionierers 4 als auch als Ausgangssignal G der Refraktärschaltung 6 gleichzeitig auf hohem Potential liegen. Die gleiche Aufgabe kann auch durch ein NOR-Gatter NOR2 wahrgenommen werden, wenn man die Signalspannung E durch einen Inverter IN1 invertiert und mit dem Signal $\bar{G}$ der Refraktärschaltung 6 vergleicht.

**Patentansprüche**

1. Synchronisierbarer Herzschrittmacher mit einem Zeitgeberkreis und einer Störerkennungsschaltung, die eine Untersuchungsstufe und eine nachfolgende Diskriminatorstufe umfaßt, in der das Ausgangssignal der Untersuchungsstufe nach einem vorgegebenen, etwa der Zeitdauer des QRS-Komplexes des Herzaktionssignals entsprechenden Zeitintervall daraufhin geprüft wird, ob es noch vorhanden ist, wobei als Ergebnis dieser Prüfung entweder das Signal als Herzsignal erkannt und an den Zeitgeberkreis weitergeleitet, oder als Störsignal erkannt und unwirksam gemacht wird, dadurch gekennzeichnet, daß die am Eingang (A) der Störerkennungsschaltung anliegende Spannung in einem Differenzierer (1) differenziert und die differenzierte Spannung der Untersuchungsstufe (2,3,4) zugeführt wird, in der der positive und der negative Spitzenwert jedes differenzierten Signals gespeichert und aus diesen Spitzenwerten die Summe gebildet wird, wobei eine negative Summenspannung bestimmter Größe ein Herzaktionssignal repräsentiert.

2. Synchronisierbarer Herzschrittmacher nach Anspruch 1, dadurch gekennzeichnet, daß der Differenzierer (1) aus einem am Eingang der Schaltung liegenden CR-Glied ($C_1$, $R_1$) besteht, das so dimensioniert ist, daß die obere Grenzfrequenz ($f_g$) etwa 1 kHz beträgt.

3. Synchronisierbarer Herzschrittmacher nach Anspruch 1 und 2, dadurch gekennzeichnet, daß die Untersuchungsstufe einen die Signalspannung anhebenden Vorverstärker (2), einen Spitzenwertspeicher (3) zum Speichern des positiven und des negativen Spitzenwertes, und eine Signalformierschaltung (4) umfaßt, in der in einem Addierer die Summe der gespeicherten Spitzenwerte gebildet und das Summensignal mit einer Referenz-Spannung verglichen wird, wobei in Abhängigkeit von dem Ergebnis dieses Vergleichs am Ausgang (E) der Signalformierschaltung (4) ein Signal mit einem hohen Pegel oder ein Signal mit einem niedrigen Pegel anliegt.

4. Synchronisierbarer Herzschrittmacher nach Anspruch 3, dadurch gekennzeichnet, daß Speicherkondensatoren ($C_3$, $C_4$) im Spitzenwertspeicher (3) über Dioden ($D_1$, $D_2$) mit unterschiedlicher Polarität aufgeladen werden.

5. Synchronisierbarer Herzschrittmacher nach Anspruch 4, dadurch gekennzeichnet, daß die Speicherkondensatoren ($C_3$, $C_4$) im Spitzenwertspeicher (3) über komplementäre Transistoren ($T_1$, $T_2$) in Emitterschaltung aufgeladen, und über weitere komplementäre Transistoren ($T_3$, $T_4$) mit umgekehrter Polarität in Emitterschaltung abgegriffen werden.

6. Synchronisierbarer Herzschrittmacher nach einem der Ansprüche 4 oder 5, dadurch gekennzeichnet, daß die Speicherkondensatoren ($C_3$, $C_4$) im Spitzenwertspeicher (3) innerhalb der Kippzeit eines monostabilen Multivibrators (MF2) der Refraktärschaltung (6) auf definierte Weise entladen werden.

7. Synchronisierbarer Herzschrittmacher nach einem der Asprüche 4 bis 6, dadurch gekennzeichnet, daß die Speicherkondensatoren ($C_3$, $C_4$) im Spitzenwertspeicher (3) über Widerstände ($R_8$, $R_9$; $R_5$, $R_6$) entladen werden.

8. Synchronisierbarer Herzschrittmacher nach einem der Ansprüche 4 bis 7, dadurch gekennzeichnet, daß die Speicherkondensatoren ($C_3$, $C_4$) im Spitzenwertspeicher (3) über analoge Schalter (AS1, AS2) oder über Feldeffekttransistoren schnell entladen werden.

9. Synchronisierbarer Herzschrittmacher nach einem der Ansprüche 1 bis 8, dadurch gekennzeichnet, daß die Auswertung des von der Untersuchungsstufe kommenden Signals in der aus einem Verzögerungsglied (5), einer Refraktärschaltung (6) und einer Koinzidenzschaltung (7) bestehenden Diskriminatorstufe in digitaler Technik erfolgt.

10. Synchronisierbarer Herzschrittmacher nach einem der Ansprüche 6 bis 9, dadurch gekennzeichnet, daß der monostabile Multivibrator (MF2) der Refraktärschaltung (6) nur nach einem als Herzsignal erkannten Signal über die volle Kippzeit wirksam ist, und nach einem als Störsignal erkannten Signal unmittelbar ein neues Refraktärzeitintervall beginnt.

**Claims**

1. A synchronizable cardiac pace maker comprising a timer circuit and a fault recognizing circuit, which has an investigation stage and a subsequent discriminator stage, in which, after a predetermined time interval corresponding approximately to the duration of the QRS complex of the cardiac action signal, the output signal of the investigation stage is examined to detect whether it is still in existence, the result of this examination either recognizing the signal as the cardiac signal and passing it on to the timer circuit, or recognizing it as a fault signal, characterized in that the voltage present at the input (A) of the fault recognition circuit is differentiated in a differentiating means (1) and the differentiated voltage is supplied to the investigation stage (2, 3 and 4), in which the positive and the negative peak value of each differentiated signal is stored and the sum of such peak values is derived, and a negative sum voltage of a certain size represents a cardiac action signal.

2. The synchronizable cardiac pace maker as claimed in claim 1 characterized in that the differentiating means (1) consists of a CR member ($C_1$ and $R_1$) connected with the input of the circuit which is so dimensioned that the upper limit frequency ($f_g$) amounts to approximately 1 kHz.

3. The synchronizable cardiac pace maker as claimed in claims 1 and 2 characterized in that the investigation stage comprises a preamplifier (2) enhancing the signal voltage, a peak value memory (3) for storing the positive and the negative peak value, and a signal shaper circuit (4), in which in an adder the sum of the stored peak values is derived and the sum signal is compared with a reference voltage, and in a way dependent on the result of this comparison, a signal with a high level or a signal with a low level is caused to appear at the output (E) of the signal shaper circuit (4).

4. The synchronizable cardiac pace maker as claimed in claim 3 characterized in that storage capacitors ($C_3$ and $C_4$) in the peak value memory (3) are charged via diodes ($D_1$ and $D_2$) with different polarities.

5. The synchronizable cardiac pace maker as claimed in claim 4 characterized in that the storage capacitors ($C_3$ and $C_4$) in the peak value memory (3) are charged via complementary transistors ($T_1$ and $T_2$) in a common emitter circuit and are tapped via further complementary transistors ($T_3$ and $T_4$) with opposite polarity in a common emitter circuit.

6. The synchronizable cardiac pace maker as claimed in claim 4 or claim 5 characterized in that the storage capacitors ($C_3$ and $C_4$) in the peak value memory (3) are discharged within the sweep time of a monostable multivibrator (MF2) of the refractory circuit (6) in a defined manner.

7. The synchronizable cardiac pace maker as claimed in one of the claims 4 through 6 characterized in that the storage capacitors ($C_3$ and $C_4$) in the peak value memory (3) are discharged via resistors ($R_8$, $R_9$; $R_5$ and $R_6$).

8. The synchronizable cardiac pace maker as claimed in one of the claims 4 through 7 characterized in that the storage capacitors ($C_3$ and $C_4$) in the peak value memory (3) are rapidly discharged via analog switches (AS1 and AS2) or via FET's.

9. The synchronizable cardiac pace maker as claimed in any one of the claims 1 through 8 characterized in that the processing of the signal coming from the investigation stage takes place digitally in a discriminator stage made up of a delay member (5), a refractory circuit (6) and a coincidence circuit (7).

10. The synchronizable cardiac pace maker as claimed in any one of the claims 6 through 9 characterized in that the monostable vibrator (MF2) of the refractory circuit (6) only acts after a signal, recognized as a cardiac signal, lasting the full sweep time and after a signal recognized as a fault signal immediately commences a new refractory time interval.

**Revendications**

1.- Stimulateur cardiaque synchronisable comportant un circuit d'horloge et un circuit de reconnaissance d'interférences qui comprend un étage d'investigation suivi d'un étage discriminateur, dans lequel le signal de sortie de l'étage d'investigation est vérifié, après un intervalle de temps prédéfini correspondant à peu près à la durée du complexe QRS du signal d'action du coeur, pour constater s'il est encore présent, étant entendu qu'à titre de résultat de cette vérification, le signal est soit reconnu comme signal du coeur et est acheminé vers le circuit d'horloge, soit reconnu comme signal perturbateur et rendu invalide, caractérisé en ce que la tension appliquée à l'entrée (A) du circuit de reconnaissance d'interférences est différencié dans un différenciateur (1) et la tension différenciée est appliquée à l'étage d'investigation (2, 3, 4), dans lequel la valeur de crête positive et la valeur de crête négative de chaque signal différencié est stockée et la somme de ces valeurs de crête est formée, une tension de somme négative de valeur déterminée représentant un signal d'action du coeur.

2.- Stimulateur cardiaque synchronisable suivant la revendication 1, caractérisé en ce que le différenciateur (1) est constitué d'un élément CR ($C_1$, $R_1$) disposé à l'entrée du circuit qui est dimensionné de telle manière que la fréquence limite supérieure ($f_g$) soit d'environ 1 kHz.

3.- Stimulateur cardiaque synchronisable suivant les revendications 1 et 2, caractérisé en ce que l'étage d'investigation comprend un préamplificateur (2) qui élève la tension du signal, une mémoire de valeurs de crête (3) pour stocker la valeur de crête positive et la valeur de crête négative et un circuit formateur de signal (4), dans lequel la somme des valeurs de crête est formée dans un additionneur et le signal de somme est comparé à une tension de référence, et en fonction du résultat de cette comparaison, un signal présentant un niveau haut ou un signal présentant un niveau bas est présent à la sortie (E) du circuit formateur de signal (4).

4. Stimulateur cardiaque synchronisable suivant la revendication 3, caractérisé en ce que des condensateurs de stockage (C3, C4) dans la mémoire de valeurs de crête (3) sont chargés par l'intermédiaire de diodes ($D_1$, $D_2$) de polarités différentes.

5. Stimulateur cardiaque synchronisable suivant la revendication 4, caractérisé en ce que les condensateurs de stockage ($C_3$, $C_4$) dans la mémoire de valeurs de crête (3) sont chargés par l'intermédiaire de transistors complémentaires ($T_1$, $T_2$) formant un montage à émetteur commun et sont déchargés par l'intermédiaire d'autres transistors complémentaires (T3, T4) de polarité inversée formant un montage à émetteur commun.

6.- Stimulateur cardiaque synchronisable suivant l'une ou l'autre des revendications 4 et 5, caractérisé en ce que les condensateurs de stockage ($C_3$, $C_4$) sont déchargés d'une manière définie dans la mémoire de valeurs de crête (3) pendant le temps de basculement d'un multivibrateur monostable (MF2) du circuit réfractaire (6).

7.- Stimulateur cardiaque synchronisable suivant l'une quelconque des revendications 4 à 6, caractérisé en ce que les condensateurs de stockage ($C_3$, $C_4$) sont déchargés dans la mémoire de valeurs de crête (3) par l'intermédiaire de résistances ($R_8$, $R_9$, $R_5$, $R_6$).

8.- Stimulateur cardiaque synchronisable suivant l'une quelconque des revendications 4 à 7, caractérisé en ce que les condensateurs de stockage ($C_3$, $C_4$) sont déchargés dans la mémoire de valeurs de crête (3) rapidement par l'intermédiaire de commutateurs analogiques (AS1, AS2) ou de transistors à effet de champ.

9.- Stimulateur cardiaque synchronisable suivant l'une quelconque des revendications 1 à 8, caractérisé en ce que l'évaluation du signal provenant de l'étage d'investigation s'effectue en technique numérique dans l'étage discriminateur constitué d'un élément de retardement (5), d'un circuit réfractaire (6) et d'un circuit à coïncidence (7).

10.- Stimulateur cardiaque synchronisable suivant l'une quelconque des revendications 6 à 9, caractérisé en ce que le multivibrateur monostable (MF2) du circuit réfractaire (6) n'est actif sur la totalité de la période de basculement qu'après un signal reconnu comme signal du coeur, tandis qu'après un signal reconnu comme signal de perturbation, un nouvel intervalle de période réfractaire débute immédiatement.

0 104 452

Fig. 1:

Fig. 2:

1

**Fig. 3:**

$$f_g = 1\,KHz$$
$$R_1 = 100\,K\Omega,$$
$$C_1 = 1,5\,nF$$

**Fig. 4:**

$$OP1 = 8021$$
$$R_2 = 22\,K\Omega$$
$$R_3 = 1\,K\Omega$$
$$C_2 = 5\,nF$$
$$f_g = 1,5\,KHz$$

**Fig. 5:**

$$R_4 = 3,3\,M\Omega$$
$$R_5 = 220\,K\Omega$$
$$R_6 = 220\,K\Omega$$
$$R_7 = 3,3\,M\Omega$$
$$C_3 = 1\,uF$$
$$C_4 = 1\,uF$$
$$T_1, T_4 = BC\,557$$
$$T_2, T_3 = BC\,547$$
$$AS1, AS2 = CD\,4066$$

50 Hz-Unterdrückung: $(1 - e^{-10ms/3s}) = 3,3 \cdot 10^{-3}$

3

Fig. 6:

$R_8 = R_9 = R_{10} = 470K\Omega$, $R_{11} = R_{13} = 3,3M\Omega$, $R_{12} = 100K\Omega$, $R_{14} = 2,2M\Omega$, $R_{15} = 470K\Omega$, $R_{16} = R_{17} = 1M\Omega$, $C_5 = 33pF$
OP2 = 8021, OP3 = 8021

Fig. 7:

MF1 = CD 4098

**Fig. 8:**

$$MF2 = CD\ 4047$$
$$R_{19}C_7 = 250 \div 300\ ms$$
$$C_8 = C_9 = 1nF$$
$$R_{20} = R_{21} = 1M\Omega$$
$$R_{22} = 2,2M\Omega$$
$$NOR1 = CD\ 4572$$
$$D_3 = D_4 = 1N4148$$

$J$ = "1" : Störsignal!

**Fig. 9:**

Alle Elemente CD 4572

Wahrheitstabelle:

| E | G | $\bar{E}$ | $\bar{G}$ | H | |
|---|---|---|---|---|---|
| 0 | 0 | 1 | 1 | 0 | |
| 1 | 0 | 0 | 1 | 0 | |
| 0 | 1 | 1 | 0 | 0 | |
| 1 | 1 | 0 | 0 | 1 | Herzsignal! |

7